# EUROPEAN PATENT SPECIFICATION

(11) **EP 2 591 110 B1**
(45) Date of publication and mention of the grant of the patent: **08.04.2015**
(21) Application number: 11736494.3
(22) Date of filing: 05.07.2011
(51) Int. Cl.: C12N 15/82, C07K 14/325, A01N 63/00

(54) **CONTROL OF COLEOPTERAN INSECT PESTS**
BEKÄMPFUNG VON INSEKTENSCHÄDLINGEN DER GATTUNG COLOEPTERA
CONTRÔLE DES INSECTES NUISIBLES DE LA FAMILLE DES COLÉOPTÈRES

(30) Priority: 07.07.2010 US 362109 P
(43) Date of publication of application: 15.05.2013
(73) Proprietor: Syngenta Participations AG, Research Triangle Park, NC 27709 (US)
(72) Inventor: GRASER, Gerson, Cary North Carolina 27502 (US); BOUDREAU, Eric, Durham North Carolina 27713 (US)
(74) Representative: Syngenta International AG
(86) International application number: PCT/US2011/042932
(87) International publication number: WO 2012/006271

(56) References cited:
- WO-A1-2008/121633
- WO-A1-2010/043928
- ANONYMOUS: "Syngenta Biotechnology, Inc. Insect Resistant MIR162 Corn. OECD Unique Identifier: SYN-IR162-4. Draft Environmental Assessment", USDA, APHIS, MD, USA , December 2009 (2009-12), pages 1-41, XP002660152, Retrieved from the Internet: URL:http://www.aphis.usda.gov/brs/aphisdoc s/07_25301_pea.pdf [retrieved on 2011-09-28]
- SUSAN C. MACINTOSH ET AL: "Potentiation of Bacillus thuringiensis insecticidal activity by serine protease inhibitors", JOURNAL OF AGRICULTURAL AND FOOD CHEMISTRY, vol. 38, no. 4, 1 April 1990 (1990-04-01), pages 1145-1152, XP055008392, ISSN: 0021-8561, DOI: 10.1021/jf00094a051
- Y. PARK ET AL: "Enhancement of Bacillus thuringiensis Cry3Aa and Cry3Bb Toxicities to Coleopteran Larvae by a Toxin-Binding Fragment of an Insect Cadherin", APPLIED AND ENVIRONMENTAL MICROBIOLOGY, vol. 75, no. 10, 15 May 2009 (2009-05-15), pages 3086-3092, XP055008309, ISSN: 0099-2240, DOI: 10.1128/AEM.00268-09
- EFSA Panel on Genetically modified Organisms: "Scientific Opinion on application (EFSA-GMO-UK-2007-50) for the placing on the market of insect resistant and herbicide tolerant genetically modified maize Bt11 x MIR604for food and feed uses, import and processing under Regulation (EC) No 1829/2003 from Syngenta Seeds.", EFSA journal, vol. 8, no. 5:1614 18 May 2010 (2010-05-18), pages 1-30, XP002660235, DOI: 10.2903/j.efsa.2010.1614 Retrieved from the Internet: URL:http://www.efsa.europa.eu/en/efsajourn al/doc/1614.pdf [retrieved on 2011-09-29]
- A. H. SAYYED ET AL: "Cyt1Aa from Bacillus thuringiensis subsp. israelensis Is Toxic to the Diamondback Moth, Plutella xylostella, and Synergizes the Activity of Cry1Ac towards a Resistant Strain", APPLIED AND ENVIRONMENTAL MICROBIOLOGY, vol. 67, no. 12, 1 December 2001 (2001-12-01), pages 5859-5861, XP055008452, ISSN: 0099-2240, DOI: 10.1128/AEM.67.12.5859-5861.2001
- BRAVO A ET AL: "How to cope with insect resistance to Bt toxins?", TRENDS IN BIOTECHNOLOGY, ELSEVIER PUBLICATIONS, CAMBRIDGE, GB, vol. 26, no. 10, 1 October 2008 (2008-10-01), pages 573-579, XP025406825, ISSN: 0167-7799, DOI: 10.1016/J.TIBTECH.2008.06.005 [retrieved on 2008-08-14]
- FRANKENHUYZEN ET AL: "Insecticidal activity of Bacillus thuringiensis crystal proteins", JOURNAL OF INVERTEBRATE PATHOLOGY, SAN DIEGO, CA, US, vol. 101, no. 1, 1 April 2009 (2009-04-01) , pages 1-16, XP026040887, ISSN: 0022-2011, DOI: 10.1016/J.JIP.2009.02.009 [retrieved on 2009-03-06]
- SCHNEPF E ET AL: "BACILLUS THURINGIENSIS AND ITS PESTICIDAL CRYSTAL PROTEINS", MICROBIOLOGY AND MOLECULAR BIOLOGY REVIEWS, AMERICAN SOCIETY FOR MICROBIOLOGY, US, vol. 62, no. 3, 1 September 1998 (1998-09-01), pages 775-806, XP000986964, ISSN: 1092-2172
- PARDO-LOPEZ L ET AL: "Strategies to improve the insecticidal activity of Cry toxins from Bacillus thuringiensis", PEPTIDES, ELSEVIER, AMSTERDAM, vol. 30, no. 3, 1 March 2009 (2009-03-01), pages 589-595, XP025991581, ISSN: 0196-9781, DOI: 10.1016/J.PEPTIDES.2008.07.027 [retrieved on 2008-08-19]
- GUANGJUN WANG ET AL: "Engineered Bacillus thuringiensis GO33A with broad insecticidal activity against lepidopteran and coleopteran pests", APPLIED MICROBIOLOGY AND BIOTECHNOLOGY, SPRINGER, BERLIN, DE, vol. 72, no. 5, 28 March 2006 (2006-03-28) , pages 924-930, XP019441674, ISSN: 1432-0614, DOI: 10.1007/S00253-006-0390-X
- S. OKAY ET AL: "Expression of chitinase A (chiA) gene from a local isolate of Serratia marcescens in Coleoptera-specific Bacillus thuringiensis", JOURNAL OF APPLIED MICROBIOLOGY, vol. 0, no. 0, 1 January 2008 (2008-01-01) , XP055008346, ISSN: 1364-5072, DOI: 10.1111/j.1365-2672.2007.03570.x
- C. ZHU ET AL: "Vegetative insecticidal protein enhancing the toxicity of Bacillus thuringiensis subsp kurstaki against Spodoptera exigua", LETTERS IN APPLIED MICROBIOLOGY, vol. 42, no. 2, 1 February 2006 (2006-02-01), pages 109-114, XP055007787, ISSN: 0266-8254, DOI: 10.1111/j.1472-765X.2005.01817.x
- PORCAR M ET AL: "Effects of Bacillus thuringiensis Cry1Ab and Cry3Aa endotoxins on predatory Coleoptera tested through artificial diet-incorporation bioassays", BULLETIN OF ENTOMOLOGICAL RESEARCH, LONDON, GB, vol. 100, no. 3, 1 June 2010 (2010-06-01), pages 297-302, XP008143358, ISSN: 0007-4853, DOI: 10.1017/S0007485309990290 [retrieved on 2009-09-28]

## Description

### FIELD OF THE INVENTION

The present invention relates generally to the control of pests that cause damage to crop plants by their feeding activities, and more particularly to the control of coleopteran pests by compositions comprising synergistic levels of a coleopteran-active protein toxin and a lepidopteran-active protein toxin. The invention further relates to the compositions and methods employing such compositions comprising the protein toxins.

### BACKGROUND

Coleopteran insects are considered some of the most important pests to crop plants. For example, species of corn rootworm are the most destructive corn pests causing an estimated loss of over $1 billion annually. Important corn rootworm pest species include *Diabrotica virgifera virgifera,* the western corn rootworm; *D. longicornis barberi,* the northern corn rootworm, *D. undecimpunctata howardi,* the southern corn rootworm, and *D. virgifera zeae,* the Mexican corn rootworm. Colorado potato beetle (CPB; *Leptinotarsa decemlineata),* is another example of a coleopteran insect which is a serious pest of potato, tomato and eggplant world-wide.

Coleopteran pests are mainly controlled by intensive applications of chemical pesticides, which are active through inhibition of insect growth, prevention of insect feeding or reproduction, or cause death. Good insect control can thus be reached, but these chemicals can sometimes also affect other, beneficial insects. Another problem resulting from the wide use of chemical pesticides is the appearance of resistant insect strains. This has been partially alleviated by various resistance management practices, but there is an increasing need for alternative pest control agents.

*Bacillus thuringiensis* (Bt) Cry proteins (also called δ-endotoxins) are proteins that form a crystalline matrix in *Bacillus* that are known to possess insecticidal activity when ingested by certain insects. Over 180 holotype Cry proteins in 58 families have been identified and named. The various Cry proteins have been classified based upon their spectrum of activity and sequence homology. Prior to 1990, the major classes were defined by their spectrum of activity (Hofte and Whitely, 1989, Microbiol. Rev. 53:242-255), but more recently a new nomenclature was developed which systematically classifies the Cry proteins based on amino acid sequence homology rather than insect target specificities (Crickmore et al. 1998, Microbiol. Molec. Biol. Rev. 62:807-813).

Genes coding for Cry proteins have been isolated and their expression in crop plants have been shown to provide another tool for the control of economically important insect pests. Such transgenic plants expressing the Cry proteins have been commercialized, allowing farmers to reduce or augment applications of chemical insect control agents. Coleopteran-active Cry proteins useful in transgenic plants include, for example, Cry3A, Cry3B and the Cry34/Cry35 complex. Examples of lepidopteran-active Cry proteins that have been expressed in transgenic plants include, for example, Cry1Ab (e.g. Cry1Aa, CrylAb, CrylAc), Cry1B, CrylF and Cry2, among others.

Another family of insecticidal proteins produced by *Bacillus* species during the vegetative stage of growth (vegetative insecticidal proteins (Vip)) has also been identified. U.S. Patents 5,877,012, 6,107,279, and 6,137,033, describe a new class of insecticidal proteins called Vip3. Other disclosures, including WO 98/18932, WO 98/33991, WO 98/00546, and WO 99/57282, have also identified homologues of the Vip3 class of proteins. Vip3 coding sequences encode approximately 88 kDa proteins that possess insecticidal activity against a wide spectrum of lepidopteran pests, including, but not limited to, black cutworm (BCW, *Agrotis ipsilon*)*,* fall armyworm (FAW, *Spodoptera frugiperda*), tobacco budworm (TBW, *Heliothis virescens),* sugarcane borer, (SCB, *Diatraea saccharalis*)*,* lesser cornstalk borer (LCB, *Elasmopalpus lignosellus*), and corn earworm (CEW, *Helicoverpa zea*), and when expressed in transgenic plants, for example corn (*Zea mays),* confer protection to the plant from insect feeding damage.

There is an ongoing need for compositions and methods for using such compositions having insecticidal activity, for instance for use in crop protection or insect-mediated disease control. Novel compositions are required to overcome the problem of resistance to existing insecticides or to prevent the development of resistance to existing transgenic plant approaches. Ideally such compositions have a high toxicity and are effective when ingested orally by the target pest. Thus any invention which provided compositions in which any of these properties was enhanced would represent a step forward in the art.

### SUMMARY

The present invention provides improved methods for control of a coleopteran insect pest which comprises applying to the locus where a coleopteran insect may feed a synergistically effective amount of at least one coleopteran-active toxin and at least one lepidopteran-active toxin, wherein the coleopteran-active protein is a modified Cry3A protein and wherein the lepidopteran-active protein is a Cry1Ab protein.

### DEFINITIONS

For clarity, certain terms used in the specification are defined and presented as follows:

"Activity" means the protein toxins and combinations of such toxins function as orally active insect control agents, have a toxic effect, or are able to disrupt or deter insect feeding, which may or may not cause death of the insect. When a composition of the invention is delivered to the insect, the result is typically death of the insect, or the insect does not feed upon the source that makes the composition available to the insect. Such a composition may be a transgenic plant expressing the toxin combinations of the invention. One example is a transgenic corn plant expressing a modified Cry3A protein and a CrylAb protein, which causes a synergistic activity against corn rootworm feeding on the transgenic corn plant.

To "control" or "controlling" insects means to inhibit, through a toxic effect, the ability of insect pests to survive, grow, feed, and/or reproduce, or to limit insect-related damage or loss in crop plants. To "control" insects may or may not mean killing the insects, although it preferably means killing the insects.

As used herein, the term "corn" means *Zea mays* or maize and includes all plant varieties that can be bred with corn, including wild maize species.

To "deliver" or "delivering" a composition or toxin means that the composition or toxin comes in contact with an insect, resulting in a toxic effect and control of the insect. The composition or toxin can be delivered in many recognized ways, e.g., orally by ingestion by the insect via transgenic plant expression, formulated protein composition(s), sprayable protein composition(s), a bait matrix, or any other art-recognized toxin delivery system.

"Effective insect-controlling amount" means that concentration of toxin or toxins that inhibits, through a toxic effect, the ability of insects to survive, grow, feed and/or reproduce, or to limit insect-related damage or loss in crop plants. "Effective insect-controlling amount" may or may not mean killing the insects, although it preferably means killing the insects.

"Expression cassette" as used herein means a nucleic acid sequence capable of directing expression of a particular nucleic acid sequence in an appropriate host cell, comprising a promoter operably linked to the nucleic acid sequence of interest which is operably linked to termination signals. It also typically comprises sequences required for proper translation of the nucleic acid sequence. The expression cassette comprising the nucleic acid sequence of interest may be chimeric, meaning that at least one of its components is heterologous with respect to at least one of its other components. The expression cassette may also be one that is naturally occurring but has been obtained in a recombinant form useful for heterologous expression. Typically, however, the expression cassette is heterologous with respect to the host, i.e., the particular nucleic acid sequence of the expression cassette does not occur naturally in the host cell and must have been introduced into the host cell or an ancestor of the host cell by a transformation event. The expression of the nucleic acid sequence in the expression cassette may be under the control of a constitutive promoter or of an inducible promoter that initiates transcription only when the host cell is exposed to some particular external stimulus. In the case of a multicellular organism, such as a plant, the promoter can also be specific to a particular tissue, or organ, or stage of development.

"Event MIR604" or "MIR604 event" or "MIR604" means a transgenic corn event, disclosed in US Patent 7,361,813, that has incorporated into its genome a *cry*3A055 transgene, disclosed in US Patent 7,230,167, and a *pmi* transgene, disclosed in US Patent No. 5,767,378. Therefore, MIR604 comprises a first transgene encoding a Cry3A055 insecticidal protein (modified Cry3A or mCry3A), useful in controlling corn rootworm (*Diabrotica spp.)* insect pests, and a second transgene encoding a phosphomannose isomerase enzyme (PMI), useful as a selectable marker, which allows a corn plant to utilize mannose as a carbon source.

"Event MIR162" or "MIR162 event" or "MIR162 event" means the transgenic corn event disclosed in International Publication No. WO 07/142840 that has incorporated into its genome a *vip3Aa20* transgene and *a pmi* transgene. Therefore, MIR162 comprises a first transgene encoding a Vip3Aa20 insecticidal protein, useful in controlling lepidopteran insect pests, and a second transgene encoding a phosphomannose isomerase enzyme (PMI), useful as a selectable marker, which allows a corn plant to utilize mannose as a carbon source.

"Event Bt11" or "Bt11 event" or "Bt11" means the transgenic corn event disclosed in US Patent 6,114,608 that has incorporated into its genome a *cry1Ab* transgene and *a pat* transgene. Therefore, Bt11 comprises a first transgene encoding a CrylAb insecticidal protein, useful in controlling lepidopteran insect pests, and a second transgene encoding a PAT enzyme, useful as a selectable marker, which confers on the corn plant herbicide tolerance.

A "gene" is a defined region that is located within a genome and that, besides the aforementioned coding nucleic acid sequence, comprises other, primarily regulatory, nucleic acid sequences responsible for the control of the expression, that is to say the transcription and translation, of the coding portion. A gene may also comprise other 5' and 3' untranslated sequences and termination sequences. Further elements that may be present are, for example, introns.

"Gene of interest" refers to any gene which, when transferred to a plant, confers upon the plant a desired trait such as antibiotic resistance, virus resistance, insect resistance, disease resistance, or resistance to other pests, herbicide tolerance, improved nutritional value, improved performance in an industrial process or altered reproductive capability. The "gene of interest" may also be one that is transferred to plants for the production of commercially valuable enzymes or metabolites in the plant.

As used herein, the term "grower" means a person or entity that is engaged in agriculture, raising living organisms, such as crop plants, for food or raw materials.

A "heterologous" nucleic acid sequence is a nucleic acid sequence not naturally associated with a host cell into which it is introduced, including non- naturally occurring multiple copies of a naturally occurring nucleic acid sequence.

A "homologous" nucleic acid sequence is a nucleic acid sequence naturally associated with a host cell into which it is introduced.

"Insecticidal" is defined as a toxic biological activity capable of controlling insects, preferably by killing them.

An "isolated" nucleic acid molecule or an isolated protein is a nucleic acid molecule or protein that, by the hand of man, exists apart from its native environment and is therefore not a product of nature. An isolated nucleic acid molecule or protein may exist in a purified form or may exist in a non-native environment such as, for example, a recombinant host cell. For example, a native Cry protein in *Bacillus thuringiensis* is not isolated, but that same Cry protein in a transgenic plant is isolated.

"Modified Cry3A (mCry3A)" means a gene or protein disclosed in US Patent No. 7,030,295, published April 18, 2006, useful in controlling corn rootworm (*Diabrotica spp.)* insect pests.

A "nucleic acid molecule" or "nucleic acid sequence" is a linear segment of single- or double-stranded DNA or RNA that can be isolated from any source. In the context of the present invention, the nucleic acid molecule or nucleic acid sequence is preferably a segment of DNA.

A "plant" is any plant at any stage of development, particularly a seed plant.

A "plant cell" is a structural and physiological unit of a plant, comprising a protoplast and a cell wall. The plant cell may be in the form of an isolated single cell or a cultured cell, or as a part of a higher organized unit such as, for example, plant tissue, a plant organ, or a whole plant.

"Plant cell culture" means cultures of plant units such as, for example, protoplasts, cell culture cells, cells in plant tissues, pollen, pollen tubes, ovules, embryo sacs, zygotes and embryos at various stages of development.

"Plant material" refers to leaves, stems, roots, flowers or flower parts, fruits, pollen, egg cells, zygotes, seeds, cuttings, cell or tissue cultures, or any other part or product of a plant.

A "plant organ" is a distinct and visibly structured and differentiated part of a plant such as a root, stem, leaf, flower bud, or embryo.

"Plant tissue" as used herein means a group of plant cells organized into a structural and functional unit. Any tissue of a plant *in planta* or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue.

"Transformation" is a process for introducing heterologous nucleic acid into a host cell or organism. In particular, "transformation" means the stable integration of a DNA molecule into the genome of an organism of interest.

"Transformed / transgenic / recombinant" refer to a host organism such as a bacterium or a plant into which a heterologous nucleic acid molecule has been introduced. The nucleic acid molecule can be stably integrated into the genome of the host or the nucleic acid molecule can also be present as an extrachromosomal molecule. Such an extrachromosomal molecule can be auto-replicating. Transformed cells, tissues, or plants are understood to encompass not only the end product of a transformation process, but also transgenic progeny thereof. A "non-transformed", "non-transgenic", or "non- recombinant" host refers to a wild-type organism, e.g., a bacterium or plant, which does not contain the heterologous nucleic acid molecule.

The "Vip3" class of proteins comprises, for example, Vip3Aa, Vip3Ab, Vip3Ac, Vip3Ad, Vip3Ae, VipAf, Vip3Ag, Vip3Ba, and Vip3Bb, and their homologues. "Homologue" means that the indicated protein or polypeptide bears a defined relationship to other members of the Vip3 class of proteins. "Vip3Aa20" (GeneBank Accession No. DQ539888) is a Vip3 homologue unique to event MIR162. It was generated by spontaneous mutations introduced into the maize-optimized *vip3Aa19* gene (GeneBank Accession No. DQ539887) during the plant transformation process.

The nomenclature used herein for DNA bases and amino acids is as set forth in 37 C.F.R. § 1.822.

### DETAILED DESCRIPTION

This invention relates to methods for synergistic coleopteran insect pest control which comprises applying to the locus where a coleopteran insect may feed a synergistically effective composition comprising at least one coleopteran-active toxin and at least one lepidopteran-active toxin. It is well known in the art that if two unrelated proteins are not toxic separately, they will not be toxic when combined. It is also known that combining a protein with no activity against a target pest with a protein active against that target pest, the non-active protein will not increase the activity of the already active protein.

According to the invention, it has now unexpectedly been found that the application of a combination of at least one coleopteran-active protein toxin and at least one lepidopteran-active protein toxin demonstrates a significant synergistic effect (i.e. the resultant coleopteran insect control is much greater than that which could be predicted from the coleopteran insect control of the coleopteran-active toxin used alone). This synergistic effect enables a commercially useful level of coleopteran insect control and helps mitigate the development of insect resistance to a single toxin.

In one embodiment, the present invention encompasses a method of controlling a coleopteran insect pest, which method comprises delivering to a coleopteran pest or environment thereof a composition comprising at least one coleopteran-active protein and at least one lepidopteran-active protein, wherein the composition controls the coleopteran pest to a greater degree than would be expected due to any individual coleopteran-active protein comprised therein alone, wherein the coleopteran-active protein is a modified Cry3A protein and wherein the lepidopteran-active protein is a Cry1Ab protein.

Examples of a Cry1Ab protein have the following GenBank Accession numbers: Cry1Ab1 (AAA22330), Cry1Ab2 (AAA22613), Cry1Ab3 (AAA22561), Cry1Ab4 (BAA00071), Cry1Ab5 (CAA28405), Cry1Ab6 (AAA22420), Cry1Ab7 (CAA31620), Cry1Ab8 (AAA22551), Cry1Ab9 (CAA38701), Cry1Ab10 (A29125), Cry1Ab11 (I12419), Cry1Ab12 (AAC64003), Cry1Ab13 (AAN76494), Cry1Ab14 (AAG16877), Cry1Ab15 (AAO13302), Cry1Ab16 (AAK55546), Cry1Ab17 (AAT46415), Cry1Ab18 (AAQ88259), Cry1Ab19 (AAW31761), Cry1Ab20 (ABB72460), Cry1Ab21 (ABS18384), Cry1Ab22 (ABW87320), Cry1Ab23 (HQ439777), Cry1Ab24 (HQ439778), Cry1Ab25 (HQ685122) and Cry1Ab26 (HQ847729). In still another embodiment, the Cry1Ab protein is that protein comprised in the Bt11 event and disclosed in US Patent 6,114,608.

In yet another aspect of this embodiment, the coleopteran pest is a Colorado potato beetle or a corn rootworm. In another embodiment, the corn rootworm is a western corn rootworm, a northern corn rootworm, a southern corn rootworm or a Mexican corn rootworm.

In another embodiment of the encompassed method, the composition is a transgenic plant expressing the coleopteran-active protein and the lepidopteran-active protein. In one aspect the transgenic plant is selected from the group consisting of soybean, cotton, rapeseed, canola, vegetables, sunflower, tobacco, tomato, sugar cane, rice, wheat, corn, rye oat, barley, turf grass and a forage crop. In another aspect, the transgenic plant is a transgenic corn plant. In yet another aspect, the transgenic corn plant is a breeding stack comprising the transgenic corn events MIR604 and Bt11. In another aspect, the transgenic corn plant is a breeding stack comprising the transgenic corn events MIR604, Bt11 and MIR162.

In another embodiment, the invention encompasses a method of controlling a corn rootworm pest, which method comprises delivering to the corn rootworm pest or an environment thereof a composition comprising a modified Cry3A (mCry3A) protein and a Cry1Ab protein, wherein the composition controls the corn rootworm pest to a greater degree than would be expected due to the mCry3A protein alone.

In another embodiment, the composition is a transgenic corn plant. In yet another embodiment, the transgenic corn plant comprises the MIR604 event and the Bt11 events.

Also disclosed is a coleopteran controlling composition comprising at least one coleopteran-active protein and at least one lepidopteran-active protein, wherein the composition controls a coleopteran pest to a greater degree than would be expected due to any individual coleopteran-active protein comprised therein alone.

The coleopteran-active protein is a modified Cry3A and the lepidopteran-active protein is Cry1Ab. Examples of a Cry1Ab protein have the following GenBank Accession numbers: Cry1Ab1 (AAA22330), Cry1Ab2 (AAA22613), Cry1Ab3 (AAA22561), Cry1Ab4 (BAA00071), Cry1Ab5 (CAA28405), Cry1Ab6 (AAA22420), Cry1Ab7 (CAA31620), Cry1Ab8 (AAA22551), Cry1Ab9 (CAA38701), Cry1Ab10 (A29125), Cry1Ab11 (I12419), Cry1Ab12 (AAC64003), Cry1Ab13 (AAN76494), Cry1Ab14 (AAG16877), Cry1Ab15 (AAO13302), Cry1Ab16 (AAK55546), Cry1Ab17 (AAT46415), Cry1Ab18 (AAQ88259), Cry1Ab19 (AAW31761), Cry1Ab20 (ABB72460), Cry1Ab21 (ABS18384), Cry1Ab22 (ABW87320), Cry1Ab23 (HQ439777), Cry1Ab24 (HQ439778), Cry1Ab25 (HQ685122) and Cry1Ab26 (HQ847729). In another aspect, the Cry1Ab protein is that protein comprised in the Bt11 event and disclosed in US Patent 6,114,608.

In yet another aspect, the coleopteran pest is a Colorado potato beetle or a corn rootworm. In another aspect, the corn rootworm is a western corn rootworm, a northern corn rootworm, a southern corn rootworm or a Mexican corn rootworm.

In one aspect, the composition is a transgenic plant expressing the coleopteran-active protein and the lepidopteran-active protein. In one aspect the transgenic plant is selected from the group consisting of soybean, cotton, rapeseed, canola, vegetables, sunflower, tobacco, tomato, sugar cane, rice, wheat, corn, rye oat, barley, turf grass and a forage crop. In another aspect, the transgenic plant is a transgenic corn plant. In another aspect, the transgenic plant is a transgenic corn plant. In another aspect, the transgenic corn plant is a breeding stack comprising the transgenic corn events MIR604 and Bt11. In another aspect, the transgenic corn plant is a breeding stack comprising the transgenic corn events MIR604, Bt11 and MIR162.

Also disclosed is a method of providing a grower with a means of controlling a coleopteran insect pest population comprising supplying or selling to the grower transgenic seed comprising a nucleic acid that encodes at least one coleopteran-active protein and at least one lepidopteran-active protein, wherein transgenic plants grown from said seed control a coleopteran pest to a greater degree than would be expected due to any individual coleopteran-active protein comprised therein alone.

The coleopteran-active protein is a modified Cry3A and the lepidopteran-active protein is Cry1Ab. Examples of a Cry1Ab protein have the following GenBank Accession numbers: Cry1Ab1 (AAA22330), Cry1Ab2 (AAA22613), Cry1Ab3 (AAA22561), Cry1Ab4 (BAA00071), Cry1Ab5 (CAA28405), Cry1Ab6 (AAA22420), Cry1Ab7 (CAA31620), Cry1Ab8 (AAA22551), Cry1Ab9 (CAA38701), Cry1Ab10 (A29125), Cry1Ab11 (I12419), Cry1Ab12 (AAC64003), Cry1Ab13 (AAN76494), Cry1Ab14 (AAG16877), Cry1Ab15 (AAO13302), Cry1Ab16 (AAK55546), Cry1Ab17 (AAT46415), Cry1Ab18 (AAQ88259), Cry1Ab19 (AAW31761), Cry1Ab20 (ABB72460), Cry1Ab21 (ABS18384), Cry1Ab22 (ABW87320), Cry1Ab23 (HQ439777), Cry1Ab24 (HQ439778), Cry1Ab25 (HQ685122) and Cry1Ab26 (HQ847729). In still another aspect, the Cry1Ab6 protein is that protein comprised in the Bt11 event and disclosed in US Patent 6,114,608.

In still another aspect, the coleopteran pest is a Colorado potato beetle or a corn rootworm. In one aspect, the corn rootworm is a western corn rootworm, a northern corn rootworm, a southern corn rootworm or a Mexican corn rootworm.

In one aspect, the transgenic plant seed and plant is selected from the group consisting of soybean, cotton, rapeseed, canola, vegetables, sunflower, tobacco, tomato, sugar cane, rice, wheat, corn, rye oat, barley, turf grass and a forage crop. In another aspect, the transgenic plant seed and plant is a transgenic corn seed and plant.

The co-expression of at least one coleopteran-active protein and at least one lepidopteran-active protein in the same transgenic plant can be achieved by genetically engineering a plant to contain and express all the genes necessary in a so called molecular stack. Alternatively, a plant, Parent 1, can be genetically engineered for the expression of certain genes encoding insecticidal proteins of the invention. A second plant, Parent 2, can be genetically engineered for the expression of certain other genes encoding insecticidal proteins of the invention. By crossing Parent 1 with Parent 2, progeny plants are obtained which express all the genes introduced into Parents 1 and 2, designated herein as a "breeding stack." Such a breeding stack to create a composition of the invention can be achieved by crossing a corn plant comprising the MIR604 event with a corn plant comprising the Bt11 event. Thus, the progeny of the breeding stack comprise a mCry3A protein and a Cry1Ab protein disclosed herein to provide a synergistic control of coleopteran insect pests.

Compositions, for example, transgenic plant seed, can also be treated with an insecticidal seed coating as described in U. S. Patent Nos. 5,849,320 and 5,876,739. Where both the insecticidal seed coating and the transgenic seed of the invention are active against the same target insect, the combination is useful (i) in a method for further enhancing activity of the synergistic composition against the target insect and (ii) in a method for preventing development of resistance to the composition by providing yet another mechanism of action against the target insect. Thus, is disclosed a method of enhancing activity against or preventing development of resistance in a target insect, for example corn rootworm, comprising applying an insecticidal seed coating to a transgenic seed. Such chemical treatments may include insecticides, fungicides or nematicides. Examples of such insecticides include, without limitation, dinotefuran, such as thiamethoxam, imidacloprid, acetamiprid, nitenpyram, nidinotefuran, chlorfenapyr, tebufenpyrad, tebuferiozide, methoxyfenozide, halofenozide, triazamate, avermectin, spinosad, fiprinol, acephate, fenamiphos, diazinon, chlorpyrifos, chlorpyrifon-methyl, malathion, carbaryl, aldicarb, carbofuran, thiodicarb, and oxamyl. Even where the insecticidal seed coating is active against a different insect, the insecticidal seed coating is useful to expand the range of insect control, for example by adding an insecticidal seed coating that has activity against lepidopteran insects to the transgenic seed, which has activity against coleopteran insects, the coated transgenic seed produced controls both lepidopteran and coleopteran insect pests.

### EXAMPLES

The invention will be further described by reference to the following detailed examples. These examples are provided for the purposes of illustration only, and are not intended to be limiting unless otherwise specified. Standard recombinant DNA and molecular cloning techniques used here are well known in the art and are described by J. Sambrook, et. al., Molecular Cloning: A Laboratory Manual, 3d Ed., Cold Spring Harbor, NY: Cold Spring Harbor Laboratory Press (2001); by T.J. Silhavy, M.L. Berman, and L.W. Enquist, Experiments with Gene Fusions, Cold Spring Harbor Laboratory, Cold Spring Harbor, NY (1984) and by Ausubel, F.M. et al., Current Protocols in Molecular Biology, New York, John Wiley and Sons Inc., (1988), Reiter, et al., Methods in Arabidopsis Research, World Scientific Press (1992), and Schultz et al., Plant Molecular Biology Manual, Kluwer Academic Publishers (1998).

### Example 1. Interaction between toxins against Colorado potato beetle

In this example the effects of mCry3A on the toxicity of CrylAb, and the effects of Cry1Ab on the toxicity of mCry3A, are measured with bioassays testing the insecticidal proteins alone and in combination. Event Bt11 corn and Event MIR604 corn express the insecticidal proteins Cry1Ab and modified Cry3A (mCry3A), respectively. Cry1Ab is active against certain Lepidoptera, whereas mCry3A is active against some species of Coleoptera. In the United States (US), the main use of Bt11 corn is for control of European corn borer (*Ostrinia nubilalis;* ECB) and the principal targets of MIR604 maize are western com rootworm *(Diabrotica virgifera virgifera;* WCR) and northern com rootworm *(Diabrotica longicornis barberi;* NCR). Through conventional breeding stacks of Bt11 and MIR604 plants, stacked Bt11 x MIR604 maize hybrids producing both Cry1Ab arid mCry3A were created. These maize hybrids provide control of ECB as well as WCR and NCR.

The indicator organisms used are first-instar ECB, which is highly sensitive to Cry1Ab, and first-instar Colorado potato beetle *(Leptinotarsa decemlineata;* CPB), which is highly sensitive to mCry3A. ECB is not sensitive to mCry3A, and CPB is insensitive to Cry1Ab. Although CPB is not a target pest of MIR604 orBt11 x MIR604 maize, it is more amenable to laboratory testing than the rootworm species targeted by mCry3A. Both ECB and CPB larvae are readily bioassayed using standard artificial diets under the same laboratory conditions. Because first instars of these species are highly susceptible to either Cry1Ab or mCry3A, the ability to detect many significant changes in the toxicity of either protein is maximized. In each combinatorial bioassay, each sensitive species is exposed to a high and a low concentration of the toxin, represented by the LC70 and LC30, respectively, in combination with a high concentration of the non-toxin, represented by the LC90 to the corresponding sensitive species.

Various experimental designs are available for testing interactions between toxins. The design depends on the model used to predict the effects of mixtures of toxins, without interaction, from effects of the compounds alone; an interaction is detected when observed effects of the mixture differ from predictions of the model. When toxins have similar effects, so that one compound can be substituted as a constant proportion of the other, the null model is called similar joint action. When this model applies, a test for interaction determines a dose response for a fixed ratio of the compounds (e.g., Tabashnik, 1992). When toxins act independently (different modes of action), the best model is independent joint action, and a test for interaction examines the effects of varying proportions of the compounds in a factorial design (e.g., Tajima *et al.,* 2002). Comprehensive data sets for Cry1Ab and mCry3A indicate that organisms sensitive to one protein will not be sensitive to the other protein; in other words, only one compound is toxic to a particular organism and the null hypothesis is that the mixture has no additional effect. In these situations, an interaction is shown by a difference between the toxicity of protein A alone and its toxicity in the presence of protein B. For an organism sensitive to protein A, in effect there is no dose-response to protein B, and hence there is no reason to expect the concentration of protein B to affect the toxicity of protein A. Therefore, testing the effect of protein B at a fixed concentration is a simple and effective method to test for any interaction. This method most closely resembles the "simple empirical approach" described by Tabashnik (1992).

Interaction between the two insecticidal proteins is detected as a statistically significant difference between the mortality observed with the single toxic protein and the mortality observed when the second protein is added in combination with the toxic protein. First-instar ECB are exposed to Cry1Ab at the LC30 and LC70, alone and in combination with a high concentration of mCry3A, corresponding to the LC90 for first-instar CPB. Correspondingly, first-instar CPB are exposed to mCry3A at the LC30 and LC70 alone and in combination with a high concentration of Cry1Ab6, corresponding to the LC90 for first-instar ECB.

Exposing the sensitive species at both their LC30 and LC70 allows evaluation of potential interaction with the second protein at two distinct points in the dose-response curve. Exposure to the second protein at a concentration that is highly toxic (LC90) to the sensitive species provides a sufficiently high exposure to detect any biologically relevant toxicity if there is interaction between the two proteins.

The sources of Cry1Ab and mCry3A used in the bioassays are test substances produced by over-expressing each protein in recombinant *E. coli* followed by purification. Cry1Ab and mCry3A as contained in these test substances are substantially equivalent to the insecticidal proteins as expressed in Bt11 and MIR604 transgenic corn plants, respectively. The use of purified proteins produced in microbes is preferable to using plant-derived preparations of Cry1Ab and mCry3A. The relatively higher purity of the microbially derived test substances allowed for more precise toxicity determinations, without interference from plant substances. These plant substances might not be present in equal quantities in both Bt11- and MIR604-derived materials, as well as in control materials, and may confound interpretation of the bioassay results.

Production of Cry1Ab test substance: The Cry1Ab test substance is determined to contain approximately 127 µg Cry1Ab/ml test substance (0.0127% w/v). After preparation, the test substance is stored at approximately 4°C. The trypsin-truncated Cry1Ab in the test substance corresponds approximately to the truncated Cry1Ab encoded in Bt11 corn. The truncated CrylAb protein encoded in Bt11 corn represents the first 615 N-terminal amino acids of the full-length native Cry1Ab protein from *B. thuringiensis* subsp. *kurstaki.* By comparison, the predominant form of trypsin-truncated Cry1Ab in the test substance is a 587-amino acid protein, representing the same truncated Cry1Ab protein present in Bt11 corn, minus the first 28 N-terminal amino acids (Kramer, 2006). Trypsinization of the Cry1Ab in Bt11 corn removes these 28 N-terminal amino acids (which are not required for insecticidal activity), and further demonstrates the substantial equivalence of E. coli-produced trypsin-truncated Cry1Ab and the truncated Cry1Ab produced in Bt11, as measured by SDSPAGE, Western blot analysis, N-terminal sequencing, peptide mapping, biological activity against neonate ECB larvae, and absence of detectable glycosylation. Therefore, the truncated Cry1Ab protein present in the test substance can be considered substantially equivalent to the truncated Cry1Ab protein encoded in Bt11 maize.

Production of mCry3A test substance: The mCry3A test substance is determined to contain approximately 90% mCry3A protein by weight, to have bioactivity against a sensitive coleopteran species and is shown to be substantially equivalent to mCry3A as produced in Event MIR604 corn, as assessed by various biochemical and functional parameters. After preparation, the mCry3Atest substance is stored at approximately -20°C.

Estimation of LC30, LC70 and LC90 for ECB Does-Response to Cry1Ab: The bioactivity of the Cry lAb is assessed in insect feeding assays using first-instar ECB larvae in accordance with standard methods known in the art. Briefly, the bioassays are conducted in Costar 24-well plates (Fisher Scientific, Cat. # PD 10-047 -05). The test solutions are prepared by diluting the liquid Cry1Ab test substance in 0.6 *µ*M ammonium carbonate buffer. One hundred µl of each dilution are added to 100 µl ECB diet (General Lepidopteran Diet from BioServe, Inc.; Frenchtown; NJ, USA) and mixed thoroughly. The ECB insect diet is prepared in accordance methods known in the art. Each well contains 200 µl insect diet containing concentrations of Cry1Ab ranging from 3 to 372 ng/ml diet. Each treatment consists of 24 replicate wells containing one ECB larva/well. The plates are maintained at ambient laboratory conditions with regard to temperature, lighting and relative humidity. To control bias, the larvae are randomly allocated to treatment groups. As controls, larvae are exposed to insect diet without test substance (diet alone); insect diet treated with the same buffer concentration used in applying the highest test substance concentration to the diet (100 µl of ca. 0.6 µM 50 mM NH4HC03, pH 9.25, buffer/100 ml diet); and diet treated with a solution of heat-inactivated Cry1Ab test substance (30 minutes at 100°C) at a concentration equivalent to the highest test substance concentration (372 ng Cry1Ab/ml diet) used in the bioassay. The heat-inactivated protein treatment serves as a control for the potential effects of added protein in the insect diet and impurities (i.e., non-Cry1Ab components) in the test substance. Mortality is assessed at around 144 hrs.

The US EPA Probit Analysis Program, version 1.5, US EPA, 1992, is used to determine LC50 and LC90 values; in addition, the slope equation for the regression of the log-dose pro bit relationship was used to determine the LC30 and LC70 values in conjunction with a normal distribution probit table (Geigy Scientific Tables, Lentner, 1982). Other probit programs can also be used.

Estimation of LC30, LC70 and LC90 for CPB Dose-Response to mCry3A: Using the mCry3A test substance, the LC30, LC70 and LC90 of mCry3A to first-instar CPB is determined in the same manner as that described above for ECB using standard methods known in the art. The test solutions are prepared by dissolving the lyophilized test substance in MilliQ® water. One hundred µg of each dilution are added to 100 µg CPB diet (BioServe, Inc., Frenchtown, New Jersey, USA) and mixed thoroughly. The CPB insect diet is prepared using methods known in the art. Each well contains 200 µl insect diet with concentrations of mCry3A ranging from 0.01 to 5 µg/ml diet. As controls, larvae are exposed to insect diet without test substance added (diet alone), insect diet treated with the same volume of MilliQ water used in applying the test substance solution to the diet alone, and diet treated with a solution of heat-inactivated mCry3A protein from the test substance (30 minutes at 100°C) at a concentration equivalent to the highest test substance concentration (5 µg mCry3A/ml diet) used in the bioassay. Mortality is assessed at 96 hrs.

Evaluation of Effect of mCry3A on Cry1Ab Toxicity: The effect of mCry3A on the toxicity of Cry1Ab is measured by exposing first-instar ECB to the LC30 (equivalent to 27 ng Cry1Ab/ml diet) and LC70 (equivalent to 70 ng Cry1Ab/ml diet) of Cry1Ab and comparing the mortality in the presence and absence of mCry3A. The concentration of mCry3A corresponding to the CPB LC90 (equivalent to 2.4 µg mCry3A/ml diet) is determined as described above.

The interaction bioassay is performed using the same culture procedures and conditions described above, except that triplicate 24-well culture plates are used for each treatment. Each treatment plate contains 24 larvae, for a total of 72 larvae per treatment. As controls, larvae are exposed to insect diet without test substance (diet alone); insect diet treated with the same buffer concentration used in applying the highest test substance concentration to the diet (100 µl of ca. 0.6 µM 50 mM NH4HC03, pH 9.25, buffer/100 ml diet); diet treated with a solution of heat-inactivated Cry1Ab (30 minutes at 100°C) at a concentration equivalent to the highest Cry1Ab concentration (372 ng Cry1Ab/ml diet) used in the bioassay; and mCry3A dosed at 2.4 µg/ml diet, corresponding to the LC90 of mCry3A against CPB. CPB diet treated with the LC70 (equivalent to 1.4 µg mCry3A/ml diet) of mCry3A against CPB is used as concurrent positive control to confirm the insecticidal activity of mCry3A. Mortality is assessed after approximately 144 and 168 hours. The entire interaction bioassay with ECB is conducted twice.

Evaluation of Effect of Cry1Ab on mCry3A Toxicity: The effect of Cry1Ab on the toxicity of mCry3A is measured by exposing first-instar CPB to the LC30 (equivalent to 0.62 µg mCry3A/ml diet) and LC70 (equivalent to 1.35 µg mCry3A/ml diet) concentrations of mCry3A and comparing the mortality in the presence and absence of Cry1Ab. The concentration of Cry1Ab is the ECB LC90 (equivalent to 142 ng CrylAb/ml diet). The number of replicate treatments and the analysis of CPB mortality data are the same as described above.

The interaction bioassays are performed using the same culture procedures and conditions described above, except that triplicate 24-well culture plates are used for each treatment. Each treatment plate contains 24 larvae, for a total of 72 larvae per treatment. As controls, larvae are exposed to insect diet without test substance (diet alone), insect diet treated with the same volume of MilliQ water used in applying the test substance solution to the diet alone, diet treated with a solution of heat-inactivated Cry1Ab (30 minutes at 100°C) at a concentration equivalent to the highest mCry3A concentration (5 *µ*g mCry3A/ml diet) used in the bioassay, and Cry1Ab dosed at 142 ng/ml diet corresponding to the LC90 of Cry1Ab against ECB. ECB diet treated with the LC70 concentration (equivalent to 70 ng Cry1Ab/ml diet) of Cry1Ab against ECB is used as concurrent positive control to confirm the insecticidal activity of Cry1Ab used in the combinatorial bioassay. Mortality is assessed after approximately 72 and 96 hours. The entire interaction bioassay with ECB is conducted twice.

Statistical Methods: In each study, several criteria have to be met for the experimental design and data analysis described below to be a valid and effective test for an interaction between the proteins: (1) There is no effect of the buffer used to dissolve the proteins; (2) There is no effect of the addition of protein *per se;* or (3) The "non-toxin" is not toxic to the insensitive bioassay species at the concentrations used in this experiment. For both the ECB and CPB experiments, these criteria are met.

Once mortality is observed in the bioassays, mortality is recorded each day until there is approximately 30% mortality in the LC30 treatment and 70% mortality in the LC70 treatment. Data from each of the sampling occasions within each protein interaction study are analyzed separately. At each sampling, separate analyses are performed for each assay alone and for the combined data from both assays.

At each sampling in each bioassay, the response analyzed is the arcsine square-root transformed proportion of dead larvae per replicate. The effects of the various treatments are tested by ANOVA. For both experiments (ECB and CPB), the two assays are analyzed separately and combined (if valid). A crucial assumption of ANOVA is that there is homogeneity of variance among the treatments and the residuals are normally distributed. This is unlikely to be true if the negative control treatments are included, as the proportion of dead larvae is zero in many replicates. This is a particular problem for ANOVA of arcsine square-root-transformed data. Therefore the negative control data are excluded from the analysis, as their validation of the assumptions of the method is clear without statistical analysis. For the assays analyzed separately, ANOVA with an effect for treatment is performed. Levene's test (SAS, 2002-2003) is used to check the assumption of homogeneity of variance within each of the four treatments and Shapiro-Wilks' test (SAS, 2002-2003) is used to check the assumption of normally distributed residuals. For analysis of the combined assays, ANOVA with effects for assay and treatment is performed. Shapiro-Wilks' test is used to check the assumption of normally distributed residuals. No formal test of the assumption of homogeneity of variances is performed, since Levene's test cannot be applied if there is more than one effect in the analysis of variance (SAS, 2002-2003). However, visual comparison of plots of the arcsine square-root-transformed data is used to confirm that the homogeneity of variance assumption was valid for the combined data.

The factorial structure of the treatments allows three effects to be investigated: (1) The main effect of the toxic protein (Does the concentration of the toxin influence the response?); (2) The main effect of the non-toxic protein (Does the presence of the non-toxin influence the response?) and (3) The interaction between the concentration of the toxin and the presence of the non-toxin (Does the effect of the non-toxin depend on the concentration of the toxin, and does the effect of toxin concentration depend on the presence of the non-toxin?)

The effects are investigated by setting up treatment contrasts that focuses on that effect while removing the other effects. This is achieved by examining appropriate combinations of the treatment means (a combination of treatment means is known as a contrast). Each contrast is the sum of the individual contrast coefficients multiplied by their associated treatment means. The statistical significance of each contrast can be assessed under an appropriate null hypothesis. The null hypotheses for the three items are as follows: (1) Ho: The response at low and high concentrations of the toxic protein is the same; (2) Ho: The response is the same with or without the non-toxic protein; and (3) Ho: Any effects of the toxic and non-toxic proteins act independently of each other.

Each contrast is evaluated at a 5% Type I error rate using the estimate of error from the ANOVA. Treatment-contrast 2 tests whether there is synergism (or antagonism) between the two proteins. Therefore if the null hypothesis for contrast 2 is rejected then the data provide evidence of synergism (or antagonism). Further examination of the sign of any significant contrasts determines whether there is synergism or antagonism (for example, a positive contrast 2 value implies greater mortality when the non-toxic protein is present, hence synergism).

The mean values and standard deviations of the combinatorial bioassays are calculated using Microsoft Excel®.

### RESULTS

Estimation of LC30, LC70 and LC90 for Cry1Ab against ECB: The bioactivity estimate for CrylAb against European corn borer larvae showed an LC30 of *ca.* 27, LC70 of *ca.* 70 and an LC90 of *ca.* 142 ng Cry1Ab/ml diet after 144 hours. The negative control diets showed only low mortality, with 8% for the diet-alone treatment, 4% for the buffer-treated diet, and 4% for diet treated with inactivated Cry1Ab.

Estimation of LC30, LC70 and LC90 for mCry3A against CPB: The bioactivity estimate for mCry3A against Colorado potato beetle larvae showed an LC30 of *ca.* 0.6, LC70 of *ca* 1.4 and an LC90 of *ca.* 2.4 µg mCry3A/ml diet after 96 hours. The negative control diets showed no or only low mortality with 0% for the diet alone treatment, 8% for the water treated diet, and 4% for diet treated with inactivated mCry3A.

Evaluation of Effect of mCry3A on Cry1Ab Toxicity: There was low mean mortality (7% or lower) on the diet alone, on the buffer-treated diet, on the heat-inactivated Cry1Ab-treated diet and the diet treated with mCry3A at the LC90 CPB (2.4 *µ*g mCry3A/ml diet) after 168 hours. Further, there was a clear difference between the toxin-treated diets and the negative control diets. Therefore, several necessary conditions for the experimental design to be valid were demonstrated.

In both assays after 144 hr, the mortalities in the LC30 and LC70 treatments (both with and without mCry3A) were statistically significantly below 30% and 70% respectively. Therefore, the assays were continued to reach the desired toxicity endpoints of the experiment. After 168 hr, the endpoints *(i.e.,* 30% and 70% mortality in the LC30 Cry1Ab alone and LC70 Cry1Ab alone treatments, respectively) had been reached. The 144- hr data were considered to have power to detect effects of mCry3A on Cry1Ab.

The results of the combined data demonstrated that the effects of Cry1Ab concentration and presence of mCry3A were independent at 144 hr and 168 hr (p >0.05) against European corn borer. Therefore, the effect of concentration of Cry1Ab could be tested by joint analysis of the data with and without mCry3A. Similarly, the effect of mCry3A could be tested by joint analysis of the LC30 and LC70 data.

In the combined data, there was a statistically significant effect of concentration of Cry1Ab (p <0.05). At 144 hr and at 168 hr, the effect of Cry1Ab concentration was statistically significant in the combined data. These data indicate that ECB was responding as expected to different concentrations of Cry1Ab.

In the combined data, no statistically significant effect of mCry3A on the toxicity of Cry1Ab was detected at 144 hr or at 168 hr (p >0.05). As no effect of mCry3A alone on ECB was detected in this study, these results provide no evidence for an interaction between CrylAb and mCry3A in killing or controlling European corn borer.

Evaluation of Effect of Cry1Ab on mCry3A Toxicity: For the CPB assays there was low mean mortality (4% or lower) on the diet alone, on the water-treated diet, on the heat-inactivated Cry1Ab-treated diet and the diet treated with Cry1Ab at the LC90 ECB (142 ng CrylAb/ml diet) after 96 hours. Further, there was a clear difference between the toxin-treated diets and the negative control diets. Therefore, several necessary conditions for the experimental design to be valid were demonstrated.

In both CPB bioassays, after 72 hr some mortality was observed; however, the mortalities in the LC30 and LC70 treatments were statistically significantly below 30% and 70% respectively in at least one bioassay. Therefore the assays were continued to reach the desired mortality endpoints of the experiment. After 96 hr, the endpoints (*i.e.,* 30% and 70% mortality in the LC30 mCry3A alone and LC70 mCry3A alone respectively) had been reached. The 72-hr data were considered to have power to detect effects of mCry3A on Cry1Ab. For the separate assays and the combined data, the criteria of normality and homogeneity of variance were met at both 72 hr and 96 hr.

In the combined data, the effects of mCry3A concentration and presence of Cry1Ab were independent at 72 hr and 96 hr *(p* >0.05). Therefore the effect of concentration of mCry3A could be tested by joint analysis of the data with and without Cry1Ab. Similarly, the effect of Cry1Ab could be tested by joint analysis of the LC30 and LC70 data.

In the combined data, there was a statistically significant effect of concentration of mCry3A at 72 hr and 96 hr *(p* <0.05). These data indicate that CPB was responding as expected to different concentrations of mCry3A.

In the combined data, no statistically significant effect of Cry1Ab on the toxicity of mCry3A was detected at 96 hr *(p* >0.05). However, in the combined data at 72 hr a statistically significant effect of Cry1Ab was detected (p <0.05). Therefore, an interaction between mCry3A and Cry1Ab was indicated at 72 hr in the combined data. The greater mortality in the presence of Cry1Ab indicates that the effect is synergism (as defined by Tabashnik, 1992) or potentiation (as defined by Haghdoost *et ai*., 1997). Therefore, Cry1Ab potentiates or synergizes the activity of mCry3A causing mCry3A to work faster against target coleopteran insects than would be expected with mCry3A alone. On a commercial scale, faster kill translates into less plant damage and less opportunity for coleopteran insect pests to develop resistance.

### Illustrative example 2.Interaction between toxins against corn rootworm

This example investigates whether there is an interaction, with regard to insecticidal activity, between a lepidopteran-active protein mixture comprising Cry1Ab and Vip3Aa20 ("Lep Composition"), and a coleopteran-active protein mixture comprising mCry3A ("Col Composition").

The effects of the Lep Composition on a sensitive pest species European corn borer (*Ostrinia nubilalis;* ECB) is investigated in the presence or absence of the Col Composition. First instar larvae are used to conduct the ECB diet incorporation bioassay. Percent ECB mortality is assessed at 120 hrs after infestation. An ECB dose-response curve with eight concentrations of the Lep Composition is established first. Two doses, ECB Dose 1 and ECB Dose 2, of the Lep Composition giving intermediate level of response are chosen from dose-response curve to conduct the lepidopteran-active and coleopteran-active protein interaction bioassay. ECB Dose 1 comprises about 25 ng Cry1Ab and about 12.5 ng Vip3Aa20 per ml of diet and ECB Dose 2 comprises about 50 ng Cry1Ab and about 25 ng Vip3Aa20 per ml of diet. Therefore, ECB Dose 2 has about 2X the amount of Cry1Ab and Vip3Aa20 protein as ECB Dose 1.

The results of the ECB bioassay are shown in Table 1. No statistically significant increase in percent ECB mortality is detected when WCR Dose 2 of the Col Composition is present, indicating there is no interaction between the Col Composition comprising mCry3A and the Lep Composition comprising Cry1Ab + Vip3Aa20 based upon ECB bioassay.

**Table 1. Results of ECB bioassay.**

| Treatment | Percent ECB Mortality |
|---|---|
| ECB Dose 1 | 21 |
| ECB Dose 1 + WCR Dose 2 | 22 |
| ECB Dose 2 | 36 |
| ECB Dose 2 + WCR Dose 2 | 40 |
| WCR Dose 2 | 4 |
| Lep Buffer (Neg Check) | 1 |
| Lep + Col Buffer (Neg Check) | 4 |

The effects of the Col Composition on the sensitive pest species Western corn rootworm (WCR, *Diabrotica virgifera*) are investigated in the presence or absence of the Lep Composition. First instar larvae are used to conduct the WCR diet incorporation bioassay. Percent WCR mortality is assessed at 120hrs after infestation. A WCR dose-response curve with eight concentrations of the Col Composition is established first. Two doses,WCR Dose 1 and WCR Dose 2, of the Col Composition giving intermediate level of response are chosen from dose-response curve to conduct the Lep Composition and Col Composition interaction bioassay. WCR Dose 1 comprises about 50 µg mCry3A per ml of diet and WCR Dose 2 comprises about 200 µg mCry3A per ml of diet. Therefore, WCR Dose 2 has about 4X the amount of mCry3A protein as WCR Dose 1.

The results of the WCB bioassay are shown in Table 2. The results indicate a higher percent WCR mortality when Dose 2 of the Lep Composition comprising Cry1Ab + Vip3Aa20 is present, indicating that the combination of the Lep Composition and the Col Composition kills WCR at a greater degree than would be expected due to the Col Composition by itself

**Table 2. Results of the WCR bioassay.**

| Treatment | Percent WCR Mortality |
|---|---|
| WCR Dose 1 | 17 |
| WCR Dose 1 + ECB Dose 2 | 46 |
| WCR Dose 2 | 48 |
| WCR Dose 2 + ECB Dose 2 | 65 |
| ECB Dose 2 | 1 |
| Col Buffer (Neg Check) | 1 |
| Col + Lep Buffer (Neg Check) | 1 |

It should be understood that the examples and embodiments described herein are for illustrative purposes only.

All publications and patent applications mentioned in this specification are indicative of the level of skill of those skilled in the art that this invention pertains.

### References

Haghdoost, N.R., Newman, L.M. and Johnson, E.M. (1997) Multiple chemical exposures: synergism vs. individual exposure levels. Reproductive Toxicology 11: 9-27.
MacIntosh, S.C., Stone, T.B., Sims, S.R., Hunst, P.L., Greenplate, J.T,, Marrone, P.G., Perlak, FJ., Fischoff, D.A. and Fuchs, R.L. (1990) Specificity and efficacy of purified Bacillus thuringiensis proteins against agronomically important insects. Journal of Invertebrate Pathology 56: 258-266.
SAS Software, Version 9.1 of the SAS System for Windows. Copyright (C) 2002-2003 SAS Institute Inc. SAS and all other SAS Institute Inc. product or service names are registered trademarks of SAS Institute Inc, Cary, NC, USA.
Tabashnik, B. (1992) Evaluation of synergism among Bacillus thuringiensis toxins. Applied and Environmental Microbiology 58: 3343-3346.
Tajima, 0., Schoen, E.D., Feron, VJ. and Groten, J.P. (2002) Statistically designed experiments in a tiered approach to screen mixtures of Fusarium mycotoxins for possible interactions. Food and Chemical Toxicology 40: 685-695.
US EPA (1992) EPA Probit Analysis Program version 1.5. Ecological Monitoring Research Division, Environmental Monitoring Systems Laboratory, U.S. Environmental Protection Agency, Cincinnati, OH, USA.

## Claims

1. A method of controlling a coleopteran insect pest which comprises delivering to a coleopteran pest or an environment thereof a composition comprising at least one coleopteran-active protein and at least one lepidopteran-active protein, wherein the composition controls the coleopteran pest to a greater degree than would be expected due to any individual coleopteran-active protein comprised therein alone, wherein the coleopteran-active protein is a modified Cry3A protein and wherein the lepidopteran-active protein is a Cry1Ab protein.

2. The method according to claim 1, wherein the coleopteran pest is a Colorado potato beetle or a corn rootworm.

3. The method according to claim 2, wherein the corn rootworm is selected from the group consisting of western corn rootworm, northern corn rootworm, southern corn rootworm and Mexican corn rootworm.

4. The method according to any one of claims 1 to 3, wherein the composition is a transgenic plant expressing the coleopteran-active protein and the lepidopteran-active protein.

5. The method according to claim 4, wherein the transgenic plant is a transgenic corn plant.

6. The method according to claim 5, wherein the transgenic corn plant is a breeding stack comprising the transgenic corn events MIR604 and Bt11.

7. The method according to claim 6, wherein the transgenic corn plant further comprises the transgenic corn event MIR162.

8. A method according to any one of claims 1 to 7, wherein the coleopteran insect is a corn rootworm pest, and wherein said method comprises delivering to the corn rootworm pest or an environment thereof a composition comprising a modified Cry3A (mCry3A) protein and a Cry1Ab protein, wherein the composition controls the corn rootworm pest to a greater degree than would be expected due to the mCry3A protein alone.

## Patentansprüche

1. Verfahren zum Bekämpfen eines Coleopteren-Schadinsekts, bei dem man eine Zusammensetzung umfassend mindestens ein coleopterenaktives Protein und mindestens ein lepidopterenaktives Protein an einen Coleopteren-Schädling oder eine Umwelt davon abgibt, wobei die Zusammensetzung den Coleopteren-Schädling besser bekämpft als aufgrund eines einzelnen coleopterenaktiven Proteins, das allein darin enthalten ist, zu erwarten wäre und wobei es sich bei dem coleopterenaktiven Protein um ein modifiziertes Cry3A-Protein und bei dem lepidopterenaktiven Protein um ein Cry1Ab-Protein handelt.

2. Verfahren nach Anspruch 1, wobei es sich bei dem Coleopteren-Schädling um einen Colorado-Kartoffelkäfer oder Maiswurzelbohrer handelt.

3. Verfahren nach Anspruch 2, wobei der Maiswurzelbohrer aus der Gruppe bestehend aus Westlichem Maiswurzelbohrer, Nördlichem Maiswurzelbohrer, Südlichem Maiswurzelbohrer und Mexikanischem Maiswurzelbohrer ausgewählt ist.

4. Verfahren nach einem der Ansprüche 1 bis 3, wobei es sich bei der Zusammensetzung um eine transgene Pflanze handelt, die das coleopterenaktive Protein und das lepidopterenaktive Protein exprimiert.

5. Verfahren nach Anspruch 4, wobei es sich bei der transgenen Pflanze um eine transgene Maispflanze handelt.

6. Verfahren nach Anspruch 5, wobei es sich bei der transgenen Maispflanze um ein Zucht-Stack, umfassend die transgenen Mais-Events MIR604 und Btl 1, handelt.

7. Verfahren nach Anspruch 6, wobei die transgene Maispflanze weiterhin das transgene Mais-Event MIR162 umfasst.

8. Verfahren nach einem der Ansprüche 1 bis 7, wobei es sich bei dem Coleopteren-Insekt um einen Maiswurzelbohrerschädling handelt und wobei das Verfahren umfasst, dass man eine Zusammensetzung, umfassend ein modifiziertes Cry3A (mCry3A)-Protein und ein Cry1Ab-Protein an den Maiswurzelbohrerschädling oder seine Umwelt abgibt, wobei die Zusammensetzung den Maiswurzelbohrerschädling besser bekämpft, als aufgrund des mCry3A-Proteins allein zu erwarten wäre.

## Revendications

1. Procédé de lutte contre un insecte nuisible coléoptère qui comprend l'apport à un animal nuisible coléoptère ou son environnement d'une composition comprenant au moins une protéine active vis-à-vis des coléoptères et au moins une protéine active vis-à-vis des lépidoptères, dans lequel la composition lutte contre l'animal nuisible coléoptère à un plus haut degré que celui dû à toute protéine active vis-à-vis des coléoptères individuelle comprise dans celle-ci seule auquel on s'attendrait, dans lequel la protéine active vis-à-vis des coléoptères est une protéine Cry3A modifiée et dans lequel la protéine active vis-à-vis des lépidoptères est une protéine Cry1Ab.

2. Procédé selon la revendication 1, dans lequel l'animal nuisible coléoptère est un doryphore de la pomme de terre ou une chrysomèle des racines du maïs.

3. Procédé selon la revendication 2, dans lequel la chrysomèle des racines du maïs est choisie dans le groupe constitué par la chrysomèle occidentale des racines du maïs, la chrysomèle septentrionale des racines du maïs, la chrysomèle maculée du concombre et la chrysomèle des racines du maïs du Mexique.

4. Procédé selon l'une quelconque des revendications 1 à 3, dans lequel la composition est une plante transgénique exprimant la protéine active vis-à-vis des coléoptères et la protéine active vis-à-vis des lépidoptères.

5. Procédé selon la revendication 4, dans lequel la plante transgénique est une plante de type maïs transgénique.

6. Procédé selon la revendication 5, dans lequel la plante de type maïs transgénique est un empilage de sélection comprenant les événements de maïs transgénique MIR604 et Btl 1.

7. Procédé selon la revendication 6, dans lequel la plante de type maïs transgénique comprend en outre l'événement de maïs transgénique MIR162.

8. Procédé selon l'une quelconque des revendications 1 à 7, dans lequel l'insecte coléoptère est un animal nuisible de type chrysomèle des racines du maïs, ledit procédé comprenant l'apport à l'animal nuisible de type chrysomèle des racines du maïs ou son environnement d'une composition comprenant une protéine Cry3A modifiée (mCry3A) et une protéine Cry1Ab, dans lequel la composition lutte contre l'animal nuisible de type chrysomèle des racines du maïs à un plus haut degré que celui dû à la protéine mCry3A seule auquel on s'attendrait.
